(19) Europäisches Patentamt
European Patent Office
Office européen des brevets

(11) Veröffentlichungsnummer: **0 116 127**
**B1**

(12) # EUROPÄISCHE PATENTSCHRIFT

(45) Veröffentlichungstag der Patentschrift: **15.01.86**

(51) Int. Cl.⁴: **C 07 C 43/188**, C 07 C 41/09, C 07 C 41/16, C 11 B 9/00

(21) Anmeldenummer: **83111282.6**

(22) Anmeldetag: **11.11.83**

(54) **Aliphatische Ether des Cyclododecen-2-ols, Verfahren zu ihrer Herstellung sowie ihre Verwendung zur Herstellung von Riechstoffkompositionen.**

(30) Priorität: **07.01.83 DE 3300341**

(43) Veröffentlichungstag der Anmeldung: **22.08.84 Patentblatt 84/34**

(45) Bekanntmachung des Hinweises auf die Patenterteilung: **15.01.86 Patentblatt 86/3**

(84) Benannte Vertragsstaaten: **AT CH DE FR GB LI NL**

(56) Entgegenhaltungen:
**EP-A-0 022 462**
**DE-A-2 152 016**
**DE-A-2 928 098**

**CHEMICAL ABSTRACTS, Band 92, Nr. 5, 4. Februar 1980, Seite 783, Nr. 41912s, Columbus, Ohio, USA, R. GRANDI et al.: "Base-induced decomposition of cyclic conjugated p-tolylsulfonylhydrazones: a novel route to (n 0 9) (3,5)pyrazolophanes"**

(73) Patentinhaber: **HÜLS AKTIENGESELLSCHAFT, Patentabteilung / PB 15 - Postfach 13 20, D-4370 Marl 1 (DE)**

(72) Erfinder: **Bartmann, Martin, Dr., Burgstrasse 35, D-4350 Recklinghausen (DE)**
Erfinder: **Burzin, Klaus, Dr., Wellerfeldweg 164, D-4370 Marl (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 116 127 B1

LIBER, STOCKHOLM 1986

**Beschreibung**

Einer der wichtigsten Komponenten bekannter Riechstoffkompositionen ist das aus den Blättern der in Malaysia vorkommenden Pogestemon cablin Benth. gewonnene Patchouli-Öl. Nahezu alle bekannten Duftsprays, Deodorant-Sprays und Parfüms enthalten in Anteilen zwischen 0,1 und 50% diese Geruchskomponente, die in entscheidender Weise instande ist, die Note eines Duftstoffes zu prägen.

Schon seit längerer Zeit gibt es Bemühungen, das aus mindestens 24 Inhaltstoffen bestehende Konzentrat durch einen leichter herstellbaren Stoff zu ersetzen. Die bisherigen Versuche müssen als unbefriedigend angesehen werden. Zwar wird behauptet, daß 3-Methyl-9-methylen-endo-tricyclo-(5, 2, 1, $0^{2,6}$)-dec-3-en-8-(exo)-ol "einige der typischen Geruchsnoten reproduzieren" soll (vgl. DE-OS 31 20 700). Als Ersatz für das Patchouli-Öl kommt diese komplexe Verbindung schon deshalb nicht infrage, weil die Herstellung viel zu aufwendig ist.

Aufgabe dieser Erfindung war es, leicht zugängliche Stoffe zu finden, die in der Lage sind, das natürliche Patchouli-Öl ganz oder teilweise zu ersetzen.

Es wurde jetzt gefunden, daß Ether auf der Basis von Cyclododecen-2-ol und Alkoholen der Formel ROH, wobei R ein geradkettiger oder verzweigter Alkyl- oder Alkenylrest mit 1 bis 4 C-Atomen ist, diesen Anforderungen entsprechen.

Mit ihrer intensiven, langanhaltenden, würzigen und leicht aldehydischen Note können die erfindungsgemäßen Ether natürliches Patchouli-Öl substituieren. Diese Geruchsnote ist überraschend, da die strukturell ähnlichen Ether des Cyclododecanols (US-PSS 3 281 474, 3 845 141 und DE-OS 2 928 098) und des Cyclododecylmethanols (DE-PS 2928347) durch einen holzigen bzw. holzig-ambraartigen Geruch gekennzeichnet sind und für den Einsatz von natürlichem Patchouli-Öl nicht infrage kommen.

Der Methylether des Cyclododecen-2-ols ist bereits bekannt. Er wurde im Rahmen einer Untersuchung einer italienischen Forschergruppe über die basische Zersetzung von cyclischen konjugierten p-Tosylhydrazonen gefunden (R. Grandi et al., J. Chem. Res. S 1979, 7, 246). Ein Hinweis auf etwaige Geruchseigenschaften findet sich dort nicht. Alle übrigen erfindungsgemäßen Ether sind neu.

Gegenstand der Erfindung sind ferner zwei Verfahren zur Herstellung der Ether. Die Reaktionen sind grundsätzlich bekannt; in Bezug auf die erfindungsgemäßen Ether stellen sie jedoch neue Herstellverfahren dar.

Ausgangspunkt der Synthese ist in beiden Fällen der zugrunde liegende Alkohol des ungesättigten zwölfgliedrigen Ringes, Cyclododecen-2-ol, der wiederum aus Cyclododecen durch Oxidation mit Selendioxid zugänglich ist.

Ein weiteres Verfahren zur Herstellung des Enols besteht darin, entsprechend der US-PS 2 426 264 das Epoxid des Cyclododecens über Lithiumphosphat zu leiten.

Nach dem ersten Verfahren wird Cyclododecen-2-ol durch starke Basen, wie z. B. Metallhydride, Metallamide oder Kalium-tert. -butanolat, in sein Enolat überführt. Es empfiehlt sich, ein inertes organisches Lösungsmittel, wie z. B. aliphatische oder aromatische Kohlenwasserstoffe, zu verwenden. Besonders bewährt hat sich die Umsetzung mit einem etwa 20%igen Überschuß Natriumamid in siedendem Xylol. Nach Zugabe des den Rest R einführenden Sulfonsäureesters, Alkylhalogenids oder Dialkylsulfats und etwa vierstündiger Behandlung bei 140°C wird die Reaktion durch Behandlung mit kalter NaOH-Lösung abgebrochen. Man setzt insbesondere ein Alkylbromid und -jodid ein. Auch Dimethylsulfat, kann eingesetzt werden.

Nach dem zweiten Verfahren wird ein Gemisch der Alkohole, die dem Ether zugrunde liegen, in Gegenwart von katalytischen Mengen konzentrierter Schwefelsäure erhitzt. Es ist in vielen Fällen vorteilhaft, zur Beschleunigung der Reaktion Kupfer-I-chlorid zuzusetzen. Zweckmäßigerweise setzt man den Alkohol der Formel ROH im Überschuß ein. Die Ausbeute liegt bei über 70 %.

Die Cyclododecen-2-ylether fallen nach beiden Verfahren in einer Reinheit von über 98 % als cis-trans-Isomerengemisch an. Das cis-trans-Isomerenverhältnis ist keine kritische Größe. In dieser Form sind die Ether einsetzbar. Sofern erwünscht, können sie durch eine Feindestillation weiter gereinigt werden.

Die erfindungsgemäßen Ether sind mit anderen Riechstoffen gut verträglich, sofern diese keine stark sauren Bestandteile enthalten. Ihr Anteil in Riechstoffkompositionen kann zwischen 0,5 und 50 Gewichtsprozent liegen. Aufgrund ihrer Geruchseigenschaften werden die Methyl- und Ethylether des Cyclododecen-2-ols bevorzugt.

Die Riechstoffkompositionen können direkt als Parfüm oder zur Parfümierung von Kosmetika, wie z.B. Cremes, Seifen, Lotionen, dienen. Eine weitere Verwendungsmöglichkeit ist die Geruchsverbesserung technischer Produkte, wie Wasch- und Reinigungsmittel, Desinfektionsmittel, Textilhilfsmittel.

In den folgenden Beispielen 1 bis 4 wird die Herstellung der aliphatischen Ether mit R = Methyl, Ethyl, Allyl und Isopropyl beschrieben. Die Beispiele 5 und 6 beschreiben Riechstoffkompositionen.

**Beispiel 1**

Cyclododecen -2-ylmethylether

25 g (0,65 Mol) Natriumamid wurden in 150 ml Xylol eingebracht und unter Rühren bis zum Sieden erhitzt. Innerhalb einer Stunde wurden 92 g (0, 5 Mol) Cyclododecen-2-ol, gelöst in 750 ml Xylol, in die siedende Suspension getropft. Zur Vervollständigung der Alkoholatbildung wurde weitere zwei Stunden am Rückfluß erhitzt. Danach tropfte man 44 g (0, 35 Mol) Dimethylsulfat hinzu. Das Reaktionsgemisch wurde weitere vier Stunden am Rückfluß gehalten und anschließend in eine Mischung aus Eis und 35 g Natriumhydroxid gegossen.

Die organische Phase wurde mit Wasser gewaschen, mit $Na_2SO_4$ getrocknet und danach destilliert.

Siedepunkt (0, 8 mbar): 99°C

Ausbeute: 87%

IR: 1635, 1100, 970 $cm^{-1}$

NMR ($CCl_4$): 5,75 - 5,2 δ (2H); 3,25 δ (S, 3H); 3,5 δ (1H)

Der Cyclododecen-2-ylmethylether besitzt einen würzigen, leicht aldehydischen Geruch.

**Beispiel 2**

Cyclododecen-2-ylethylether

In einem Rührkolben wurden 64 g (0,25 Mol) Cyclododecen-2-ol 250 g Ethanol und 2,5 g $Cu_2Cl_2$ gemischt. Anschließend tropfte man vorsichtig 2,5 g $H_2SO_4$ (konzentriert) hinzu und erhitzte die Reaktionsmischung für eine Stunde zum Sieden. Danach destillierte man das überschüssige Ethanol ab, nahm den Rückstand in 300 ml n-Hexan auf und wusch diese Lösung mit 10%iger Sodalösung und danach mit Wasser neutral. Nach dem Trocknen über $Na_2SO_4$ zog man das Hexan am Rotationsverdampfer ab und isolierte das Produkt durch fraktionierte Vakuumdestillation.

Siedepunkt (0,3 mbar): 90°C

Ausbeute: 90%

1R:1635, 1100 970 $cm^{-1}$

NMR ($CCl_4$): 5,75 - 5,2 δ(2H); 3,7 - 3,3 δ (m, 3H); 1,2 δ (t 3H)

Der Cyclododecen-2-ylethylether besitzt einen intensiven würzigen, leicht aldehydischen Geruch und erinnert an natürliches Patchouli-Öl.

**Beispiel 3**

Cyclododecen -2-ylallylether

Der Cyclododecen-2-ylallylether wurde entsprechend den Angaben in Beispiel 2 durch Umsetzung von 64 g (0,25 Mol) Cyclododecen-2-ol mit 313 g (5,4 Mol) Allylalkohol erhalten.

Siedepunkt (0, 1 mbar): 110 °C

Ausbeute: 72%

IR: 1635, 1100, 990, 970, 920 $cm^{-1}$

NMR ($CCl_4$): 6,0 - 5,0 δ (m, 5H); 4,05 - 3,6 δ (m, 3H)

**Beispiel 4**

Cyclododecen-2-ylisopropylether

Der Cyclododecen-2-ylisopropylether wurde entsprechend den Angaben in Beispiel 2 durch Umsetzung von 64 g (0,25 Mol) Cyclododecen-2-ol mit 324 g (5, 4 Mol) Isopropylalkohol erhalten.

Siedepunkt (0,1 mbar): 106 °C

Ausbeute: 75%

IR: 1635, 1100, 970 $cm^{-1}$

NMR ($CCl_4$): 5,75 - 5,2 δ (2H); 3. 9 - 3,6 δ (m, 2H); 1,2 δ (d, 6H)

**Beispiel 5**

Blumenartige Phantasiekomposition

Cyclododecen-2-ylethylether 100

7 -Hydroxy-6, 7-dihydrocitronellol 150

2-Phenylethanol 50

3 -Phenylpropanol 50

Ylang (synth.) 50

2, 5-Dioxa-cyclohexadecadion-(1, 6) 50

Linalool 200

Linylylacetat 150

Benzylacetat 50

Bergamotte (synth.) 50

Gemisch aus 1 -(3, 4-Epoxy-4-methylpentyl)-4- 100 und 5-formylcyclohexan

1000

**Beispiel 6**

Orientalische Duftkomposition

Cyclododecen-2-ylmethylether 100

Cumarin 30

Bergamotteöl 50

Geraniol 100

Citronellol 100

2 -Phenylethanol 120

Geraniumöl Bourbon 30

Benzoe 20

Styrax 20

Eichenmoos -Extrakt 10

2-Pentylzimtaldehyd 80

Benzylacetat 100

α -Methylionon 65

Sandelholzöl (ostindisch) 20

4-tert. -Butylcyclohexylacetat 50

Lavandinöl 50

2,5-Dioxa -cyclohexadecadion-(1, 6) 55

1000

Substanzen wie Ylang etc., deren Struktur sich nicht unmittelbar aus ihrem Namen ergibt, sind in dem Buch von St. Arctander "Perfume and Flavor Chemicals" Montclair N. Y., USA 1969, beschrieben.

**Patentansprüche:**

1. Cyclododecen-2-ylether der allgemeinen Formel

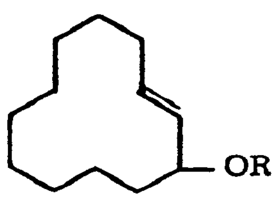

oder

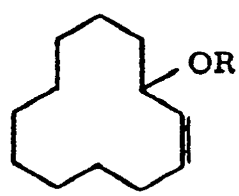

in der R ein geradkettiger oder verzweigter Alkyl- oder Alkenyl rest mit 2 bis 4 C-Atomen ist.

2. Cyclododecen-2-ylether nach Anspruch 1, wobei R einen Ethylrest bezeichnet.

3. Verfahren zur Herstellung eines Cyclododecen-2-ylethers der Formel gemäß Anspruch 1, wobei R ein geradkettiger oder verzweigter Alkyl- oder Alkenylrest mit 2 bis 4 C-Atomen ist, dadurch gekennzeichnet, daß man entweder Cyclododecen-2-ol in sein Enolat überführt und dieses anschließend mit den Rest R einführenden Sulfonsäureestern, Alkylhalogeniden oder Dialkylsulfaten umsetzt oder ein Gemisch aus Cyclododecen-2-ol und einem Alkohol der Formel ROH in Gegenwart von konzentrierter Schwefelsäure und gegebenenfalls von wasserfreiem Kupfer-I-chlorid erhitzt.

4. Verwendung der Cyclododecen-2-ylether gemäß Anspruch 3 sowie von Cyclododecen-2-ylmetylether als Riechstoffe.

5. Verwendung des Cyclododecen-2-ylmethylethers und des Cyclododecen-2-ylethylethers gemäß Anspruch 4.

6. Geruchskomposition, gekennzeichnet durch einen Gehalt von 0, 5 bis 50 Gew.-% der im Anspruch 45 genannten Cyclododecen-2-ylether.

## Claims

1. Cyclododecen-2-yl ethers of the general formula

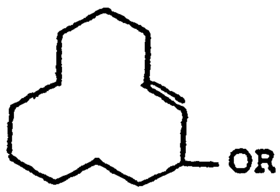

or

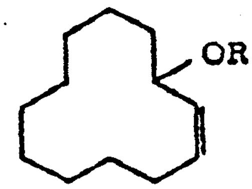

where R is straight-chain or branched alkyl or alkenyl of 2 to 4 carbon atoms.

2. Cyclododecen-2-yl ethers according to claim 1, where R is ethyl.

3. A process for the production of a cyclododecen-2-yl ether of either of the formulae in claim 1 where R is straight-chain or branched alkyl or alkenyl of 2 to 4 carbon atoms, characterised in that either cyclododecen-2-ol is converted into its enolate and this is subsequently reacted with a sulphonic acid ester, an alkyl halide or a dialkyl sulphate or a mixture of cyclododecen-2-ol and an alcohol of the formula ROH is heated in the presence of concentrated sulphuric acid and optionally waterfree cuprous chloride.

4. The use of a cyclododecen-2-yl ether according to claim 3 as well as cyclododecen-2-ylmethyl ether as a perfume.

5. The use of a cyclododecen-2-yl ether according to claim 4, where R is methyl or ethyl, as a perfume.

6. An odoriferous composition characterised by a content of 0.5 to 50% of a cyclododecen-2-yl ether according to claim 4.

## Revendications

1. Les éthers cyclododécène-2-yliques de la formule générale:

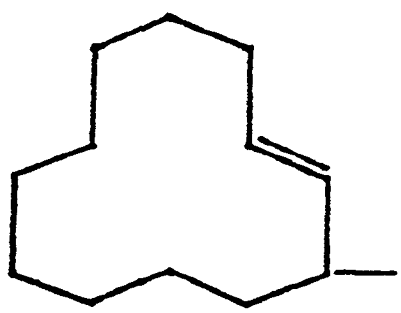

ou

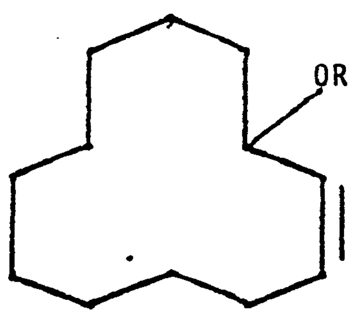

dans laquelle R est un radical alkyle ou alcényle à chaîne droite ou ramifié comportant de 2 à 4 atomes de carbone.

2. Les éthers cyclododécène-2-yliques selon la revendication 1, dans lesquels R désigne un radical éthyle.

3. Procédé pour la préparation d'un éther cyclododécène-2-ylique de la formule selon la revendication 1, dans laquelle R est un radical alkyle ou alcényle à chaîne droite ou ramifié comportant de 2 à 4 atomes de carbone, caractérisé par le fait qu'ou bien on convertit le cyclododécène-2-ol en son énolate et fait ensuite réagir celui-ci sur des esters d'acide sulfonique, des halogénures d'alkyle ou des sulfates de dialkyle, ou bien qu'on chauffe un mélange de cyclododécène-2-ol et d'un alcool de la formule ROH en présence d'acide sulfurique concentré et éventuellement de chlorure de cuivre-(I).

4. L'utilisation comme parfums des éthers cyclododécène-2-yliques selon la revendication 3 et de l'éther méthylcyclododécène-2-ylique.

5. L'utilisation comme parfums de l'éther éthylcyclo-dodécène-2-ylique selon la revendication 4, et de l'éther méthylcyclododécène-2-ylique.

6. Composition odoriférante, caractérisée par une teneur de 0,5 à 50 % en éthers cyclododécène-2-yliques selon la revendication 4.